# EUROPEAN PATENT SPECIFICATION

(11) **EP 1 641 417 B1**
(45) Date of publication and mention of the grant of the patent: **13.06.2007**
(21) Application number: 04777238.9
(22) Date of filing: 29.06.2004
(51) Int. Cl.: A61F 13/62

(54) **ELASTIC FASTENING SYSTEM**
ELASTISCHES BEFESTIGUNGSSYSTEM
SYSTEME DE FERMETURE ELASTIQUE

(30) Priority: 30.06.2003 US 610006
(43) Date of publication of application: 05.04.2006
(73) Proprietor: VELCRO INDUSTRIES B.V., Curacao (AN)
(72) Inventor: NEEB, Alexander, J., Alpharetta, GA 30022 (US); SCHMIDT, Richard, J., Roswell, GA 30076-4521 (US); PIERCE, Joseph, E., Appleton, WI 54915 (US); KURTZ, Wallace, L., Lunenburg, MA 01462 (US)
(74) Representative: Müller-Boré & Partner Patentanwälte
(86) International application number: PCT/US2004/020838
(87) International publication number: WO 2005/004778

(56) References cited:
- US-A1- 2002 016 581
- US-A1- 2002 022 108
- US-A1- 2003 034 583

## Description

### TECHNICAL FIELD

This invention relates to hook and loop fasteners.

### BACKGROUND

A number of fastening systems, such as diaper fastening systems, incorporate a hook and loop system for easy fastening and release. The hook component typically includes a flat plastic sheet laminate with a number of protruding hooks that engage with a number of loops on a corresponding loop component.

Current diaper fastening systems incorporate a non-elastic hook backing attached to a stretchable ear tab laminate. In a fastened state, this hook component is secured to a loop component on a corresponding end of the product. The loop component typically lacks any significant elastic properties as well. When the infant's body flexes, specifically when the abdomen and hip circumference increases, the fastening system responds with the elongation of the stretchable ear tab component. When the elastic tensioned response of the tab equals the ultimate shear fastening strength of the hook and loop, the product fails with a fastening "pop-open."

There is a need or desire for a hook and loop fastening system designed to provide fastening security while being stretched.

### SUMMARY

The present invention is directed to a hook and loop fastening system wherein both a hook component and a loop component can have elastic properties. The composite elastic fastening system can be designed to elongate to the wearer's maximum product straining level without significant local hook/loop failure. The elongation occurs in the elasticized backing portions of the hook and loop components.

The backing of the hook component can be an elastic nonwoven, or an elastic laminate, with the hooks protruding from the web or laminate. The hook backing can stretch in a transverse direction, or biaxially. The loop component can also be nonwoven with similar elastic properties. By providing stretch in the transverse direction of the hook portion, with strips or rows of hooks running in the longitudinal direction, the amount of energy absorbed by the system before disengaging is increased. Furthermore, pleats or hinges or creping can be located between the strips of hooks, thereby optimizing the stretch and recovery of the web. The elastic hook and loop fastening system is particularly suitable for use in absorbent articles.

With the foregoing in mind, various aspects of the invention provide a hook and loop fastening system wherein the hook component and the loop component can yield or extend, thus avoiding disengagement.

In an aspect, the invention features a hook and loop fastener that includes a hook component and a loop component. The hook component has a hook backing and a plurality of hooks protruding from the hook backing. The hook backing includes an extensible material. The loop component includes a loop backing and a plurality of loops protruding from it. The loop backing also includes an extensible material.

Implementations of this aspect may include one or more of the following features. For example, the plurality of hooks are aligned in at least two rows. The extensible material includes pleats parallel to the rows of hooks, the pleats located between the rows of hooks.

In some embodiments, the hook component further comprises a strip of material between at least some of the hooks and the hook backing. In some cases, the strips of material are co-formed with the hooks. In some cases, the strips of material can be stretchable in a longitudinal direction and the hook backing is biaxially stretchable.

In some embodiments, the hook backing and/or the loop backing includes an elastic nonwoven web.

The hook and/or loop backing, in some embodiments, includes an elastic woven web.

In some cases, the hook backing and/or loop backing includes a neck-bonded laminate.

In some embodiments, the hook backing can be stretched between about 50% and about 200%, between about 70% and about 170% and between about 100% and about 150%.

In some cases, the extensible material is creped.

In another aspect, the invention features a hook component including an extensible, sheet-form substrate having first and second regions and a multiplicity of spaced-apart resin bases disposed on a front side of the substrate. The spaced-apart resin bases have integrally molded stems extending therefrom. The bases in the first region are arranged to provide different overall extensibility than in the second region.

Implementations may include one or more of the following features. For example, the resin bases are non-contiguous with each other.

In some embodiments, the resin bases are arranged to form areas of the substrate having asymmetric extensibility.

In some cases, the bases extend in discrete, longitudinal and/or transverse rows.

The bases, in some cases, form spaced-apart patches of hooks.

In some embodiments, the extensible material is biaxially extensible having an extensibility in a first direction and a second direction, perpendicular to the first direction. In some cases, the bases are arranged to limit the level of extensibility of the extensible substrate more in one of the first and second directions than the other direction in the first region. In some cases, the bases are arranged to limit the level of extensibility of the extensible substrate in both the first and second directions in the first region.

In some embodiments, the base portions are substantially non-extensible.

The hook component, in some cases, includes at least two regions having different extensibilities.

In some embodiments, the hook backing includes an elastic nonwoven web, an elastic woven web and/or a neck-bonded laminate.

In some embodiments, the hook backing can be stretched between about 50% and about 200%, between about 70% and about 170% and between about 100% and about 150%.

In yet another aspect, the invention features a hook and loop fastener including a hook component having an extensible, sheet-form substrate having first and second regions and a multiplicity of spaced-apart resin bases disposed on a front side of the substrate. The spaced-apart resin bases have fastener elements that include stems extending outwardly from and integrally molded with the resin bases. The bases in the first region are arranged to provide different overall extensibility than in the second region. The hook and loop fastener also includes a loop component including an extensible loop backing and a plurality of loops protruding therefrom. The loops of the loop component sized to engage the fastener elements of the hook component.

Implementations may include one or more other features. For example, the extensible loop backing includes spaced-apart material disposed on a broad surface of the loop backing. In some cases the spaced-apart material disposed on the broad surface of the loop backing is arranged to provide different overall extensibility in a first region than in a second region. In some cases, fastener elements disposed in the first region of the hook component are engaged with loops in the first region of the loop component.

In some embodiments, at least one of the extensible sheet-form substrate and the extensible loop backing are biaxially extensible having an extensibility in a first direction and a second direction, perpendicular to the first direction.

In some cases, the substrate and/or the loop backing includes an elastic nonwoven web.

The sheet-form substrate and/or loop backing, in some embodiments, includes an elastic woven web.

In some cases, the sheet-form substrate and/or loop backing includes a neck-bonded laminate.

In some embodiments, the sheet-form substrate can be stretched between about 50% and about 200%, between about 70% and about 170% and/or between about 100% and about 150%.

In another aspect, the invention features a method of forming a hook and loop fastener product including providing a sheet-form extensible first substrate having first and second regions; attaching a multiplicity of spaced-apart resin bases on a front side of the substrate in the first region, the spaced-apart resin bases having fastener elements including stems extending outwardly from and integrally molded with the resin bases, to provide different overall extensibility in the first region than in the second region; and engaging the fastener elements with matable elements extending from a second substrate.

Implementations may include one or more of the following features. For example, the matable elements are loops and/or nonwoven fibers.

In some embodiments, the second substrate is extensible. In some cases, the second substrate is biaxially extensible having an extensibility in a first direction and a second direction, perpendicular to the first direction. In some cases, the method includes attaching a multiplicity of discrete, spaced-apart material on a broad surface of the second substrate in a first region to provide a different overall extensibility in the first region relative to a second region of the second substrate.

In some embodiments, the resin bases are non-contiguous with each other.

In some cases, the resin bases are arranged to form areas of the substrate having asymmetric extensibility.

Implementations of any of the foregoing aspects may include one or more of the following features. For example, the hooks include heads that are hook-shaped overhanging the hook backing in one or more discrete directions.

In some cases, the hooks include heads that are mushroom-shaped overhanging the hook backing in multiple directions.

Aspects may be included in an absorbent article, a diaper, a training pant, a feminine hygiene product, an incontinence product and/or a medical garment.

Embodiments of the invention may have one or more of the following advantages. The extensibility of the hook backing and or loop backing can be tailored to a particular application by varying the extensible material. The extensible material can be monoaxially or biaxially extensible.

In some cases, the extensibility of the extensible material can be altered/controlled by attaching a less extensible material to a surface of the extensible material. Where less extensible material is attached to the surface of the extensible material (e.g., the hook backing or substrate), the less extensible material can be arranged to limit the extensibility in a particular region more than the extensibility in another region. Also, in embodiments including a biaxially extensible material, the extensibility can be reduced more in one direction, than in another direction. By controlling the extensibility, an extensible material can be used in a wider variety of applications, for example, where less extensibility is needed, in a particular application. This includes applications where it is desirable to have an extensible fastener component that is more extensible in one region than in another region.

In some cases, arrangement of the less extensible material on the surface of the extensible material produces various stretching modes. For example, if little or no extensibility is desired in the transverse direction, transversely extending rows of less extensible material can be attached to the substrate. The transversely extending rows of less extensible material can act like beams that greatly reduce the substrate's extensibility in the transverse direction.

In some embodiments, it may also be desirable to create asymmetric extensibility within a particular region of the extensible material. For example, within a region, certain sections of the extensible material can have a different extensibility than other sections within the region by arranging the less extensible material accordingly.

In some cases, the fastening system can be designed to elongate to the wearer's maximum product straining level without significant local hook/loop failure. This is because by providing stretch in the transverse direction of the hook portion, with strips or rows of hooks running in the longitudinal direction, the amount of energy absorbed by the system before disengaging is increased.

The details of one or more embodiments of the invention are set forth in the accompanying drawings and the description below. Other features, objects, and advantages of the invention will be apparent from the description and drawings, and from the claims.

### DESCRIPTION OF DRAWINGS

Fig. 1 is a transverse view of a hook component and a loop component engaged with one another.
Fig. 2 is a longitudinal view of a hook component and a loop component engaged with one another.
Fig. 3 is a graph showing the benefits of a hook and loop fastening system having elastic properties compared to a non-elastic hook and loop fastening system.
Fig. 4 is a longitudinal view of an individual hook of a hook component having a J-shaped engagement portion.
Fig. 5 is a longitudinal view of an individual hook of a hook component having a flat-top engagement portion.
Fig. 6 is a transverse view of an individual hook of a hook component having a flat-top engagement portion.
Fig. 7 is a plan view of a diaper in a stretched flat state, showing the surface of the diaper that faces away from the wearer when the diaper is worn.
Fig. 8 is a top view of a hook component.
Fig. 9 is a side view of a hook component.
Fig. 10 is a transverse view of a hook component having pleats between rows of hooks.
Figs. 11 and 11A are top views of a hook component in unstretched and stretched conditions, respectively.
Figs. 12 and 12A are top views of another hook component in unstretched and stretched conditions, respectively.
Figs. 13 and 13A are also top views of another hook component in unstretched and stretched conditions, respectively.
Figs. 14 and 14A are also top views of another hook component in unstretched and stretched conditions, respectively.
Figs. 15 and 15A are also top views of another hook component in unstretched and stretched conditions, respectively.

Like reference symbols in the various drawings indicate like elements.

### DEFINITIONS

Within the context of this specification, each term or phrase below will include the following meaning or meanings.

"Biaxially stretchable" and "biaxial elastic stretch" refer to stretchability in two directions perpendicular to one another, e.g. stretchability in a longitudinal direction and in a transverse direction.

"Bonded" refers to the joining, adhering, connecting, attaching, entangling, embedding, or the like, of two elements. Two elements will be considered to be bonded together when they are bonded directly to one another or indirectly to one another, such as when each is directly bonded to intermediate elements.

"Creped" or "Crepe" refers to a material or composite having bonded and unbonded areas, in which the bonded areas are bent out-of-plane and the unbonded portions are looped, such that the creped material cannot be returned to its original uncreped state by applying a mechanical stress, but can be stretched or extended by applying a mechanical stress.

"Elastic," "elasticized" and "elasticity" mean that property of a material or composite by virtue of which it tends to yield to a force and recover to approximately its original size and shape after removal of a force.

"Extensible" or "stretchable" means that a material can be stretched, without breaking, by at least 50% (to at least 150% of its initial (unstretched) length) in at least one direction, suitably by at least 70% (to at least 170% of its initial length), desirably by at least 100% (to at least 200% of its initial length). The term includes elastic materials as well as materials that stretch but do not significantly retract.

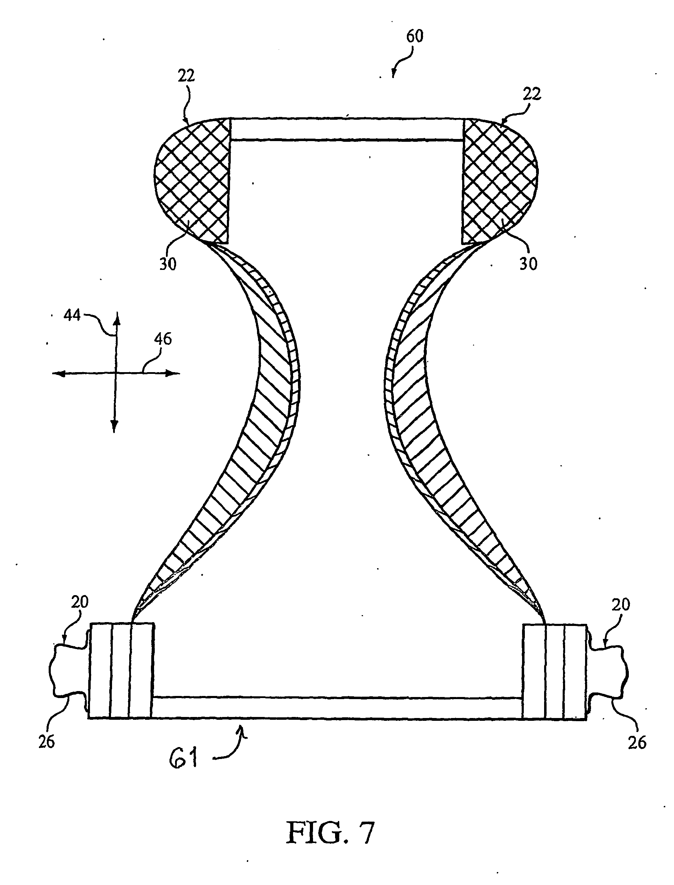

"Longitudinal" and "transverse" have their customary meaning, as indicated by the longitudinal and transverse axes depicted in Fig. 7. The longitudinal axis lies in the plane of the article and is generally parallel to a vertical plane that bisects a standing wearer into left and right body halves when the article is worn. The transverse axis lies in the plane of the article generally perpendicular to the longitudinal axis. The article as illustrated is longer in the longitudinal direction than in the transverse direction.

"Maximum product straining level" refers to a maximum amount of force, or stretching, that can be endured by a product without the product tearing or becoming completely unfastened.

"Necked" or "neck-stretched" interchangeably refer to a method of elongating a nonwoven fabric, generally in the longitudinal, or machine direction, to reduce its width in a controlled manner to a desired amount. The controlled stretching may take place under cool, room temperature or greater temperatures and is limited to an increase in overall dimension in the direction being stretched up to the elongation required to break the fabric, which in most cases is about 1.2 to 1.4 times. When relaxed, the web retracts toward its original dimensions. Such a process is disclosed, for example, in U.S. Patent Nos. 4,443,513 to Meitner and Notheis, 4,965,122, 4,981,747 and 5,114,781 to Morman and 5,244,482 to Hassenboehler Jr. et al.

"Neck bonded laminate," or NBL, conventionally refers to a composite material having at least two layers in which one layer is a necked, non-elastic layer and the other layer is an elastic layer. The layers are joined together when the non-elastic layer is in an extended condition.

"Partial failure shear tension" refers to a level of shear tension sufficient to disengage a certain fraction of hooks and loops of a hook and loop fastener.

"Peel force" refers to a force that tends to pull two adjoining bodies away from one another in opposite directions generally perpendicular to a plane in which the bodies are joined.

"Personal care garment" includes diapers, training pants, swim wear, absorbent underpants, adult incontinence products, feminine hygiene products, medical garments, and the like. The term "medical garment" includes medical (i.e., protective and/or surgical) gowns, caps, gloves, drapes, face masks, blood pressure cuffs, bandages, veterinary products, mortuary products, and the like.

"Pleats" refer to folds in material wherein the material is doubled upon itself and kept in place by pressing, stitching or any other suitable methods. The pleats allow the material to be stretched or extended when a mechanical force is applied thereto.

"Polymers" include, but are not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to isotactic, syndiotactic and atactic symmetries.

"Releasably attached," "releasably engaged" and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment.

"Shear fastening strength" refers to a fastener's ability to withstand shear tension, or shear fastening force, between corresponding fastener components.

"Shear tension" and "shear fastening force" refer to forces that tend to produce an opposite but parallel sliding motion between two bodies' planes. Shear forces tend to pull two adjoining bodies away from one another in opposite directions generally parallel to a plane in which the bodies are joined.

"Thermoplastic" describes a material that softens when exposed to heat and which substantially returns to a non-softened condition when cooled to room temperature.

These terms may be defined with additional language in the remaining portions of the specification.

### DETAILED DESCRIPTION

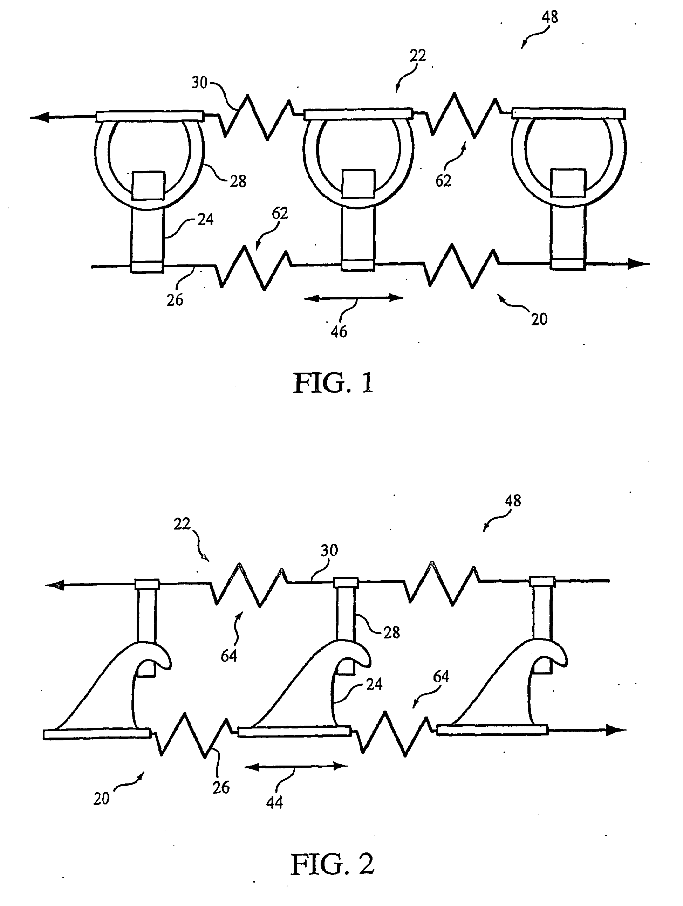

Referring to Figs. 1 and 2, a hook and loop fastening system 48 is designed to buffer or conform to product stresses during wear. The fastening system utilizes a hook component 20 and a loop component 22 each having elastic properties.

This hook and loop fastening system 48 is particularly suitable for use on disposable absorbent articles. Examples of such suitable articles include diapers, training pants, feminine hygiene products, incontinence products, other personal care or health care garments, including medical garments, or the like.

As shown in Figs. 1 and 2, a hook component 20 and a loop component 22 can be brought together to be releasably attached, or releasably engaged, to one another. The hook component 20 has a number of individual hooks 24 protruding generally perpendicularly from a hook backing 26. The hook backing 26 includes an elastic material, thereby enabling the hook component 20 to stretch in response to shear fastening force. Similarly, the loop component 22 has a number of individual loops 28 protruding generally perpendicularly from a loop backing 30, and the loop backing 30 includes an elastic material, thereby enabling the loop component 22 to stretch in response to shear fastening force.

The individual hooks 24 and the individual loops 28, when brought into contact with one another, engage with one another, with the hooks 24 latching onto the loops 28. The hooks 24 and loops 28 can be forcibly separated by pulling on either component 20, 22 using peel force. When a product deforms due to wear, the elastic properties of the hook component 20 and the loop component 22 absorb tension by elongating, thereby minimizing disengagement.

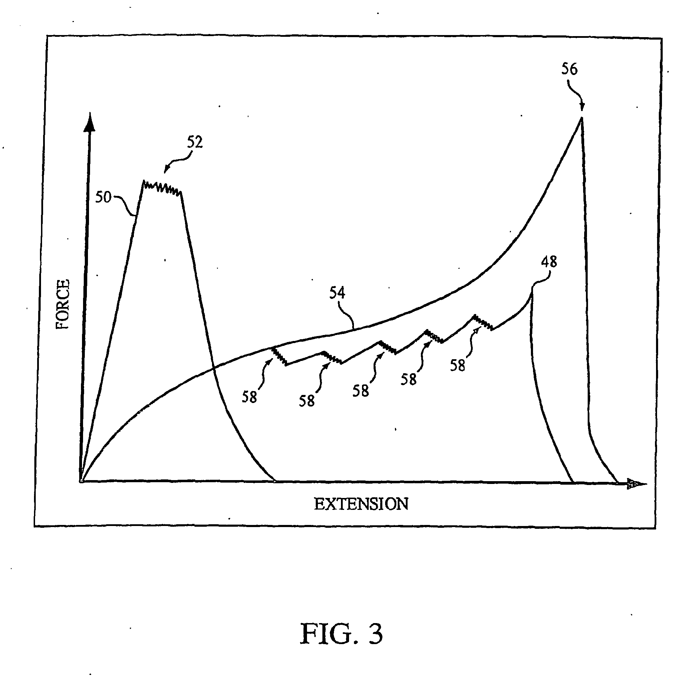

The graph in Fig. 3 shows a general comparison between a non-elastic coupling 50, a piece of elastic 54, and the integrated system 48, in terms of the distance each can extend in response to applied force. More particularly, as demonstrated by the graph in Fig. 3, the non-elastic coupling 50 between a hook component and a loop component cannot extend very far in response to applied shear force. The area designated by the reference numeral 52 shows where individual hooks or rows of hooks on the non-elastic coupling 50 become disengaged. In the non-elastic coupling 50, hook disengagement occurs all at once, thus the fastening components become unfastened after very little elongation. In contrast, the piece of elastic 54, not including a fastening component, can extend a great deal in response to applied shear force. Once the piece of elastic 54 reaches the maximum applied shear force that it can withstand, the elastic 54 breaks, as shown at point 56 in Fig. 3. The integrated system 48 is a combination of a hook component and a loop component each having elastic properties. The integrated system 48 is shown to be able to extend a great deal in response to applied shear force, relative to the non-elastic coupling 50. The areas designated by the reference numeral 58 show where individual hooks or rows of hooks become disengaged through failure shear tension, but as shown in Fig. 3, the elastic fastening system 48 is able to remain fastened even after several hooks and/or rows of hooks become disengaged, until finally, under increasing applied shear force, the integrated fastening system 48 eventually becomes completely unfastened. Furthermore, re-engagement of the hooks and loops can occur when the shear tension is decreased, such that the level of extension can move to the left along the x-axis in Fig. 3. Before the integrated fastening system 48 becomes unfastened, not only can the system 48 withstand a great deal of force, but it can also withstand considerable elongation. Therefore, when applied to an absorbent article, for example, the elastic fastening system 48 can be designed such that any foreseeably applied shear tension would not reach levels near the failure shear tension of the system 48. The shear stress foreseeably exerted on the fastening system 48 while in use is complementary to the amount of stress the fastening system 48 can withstand. In other words, the fastening system 48 includes a hook backing 26 and a loop backing 30 each having enough elasticity to elongate to a wearer's maximum product straining level without significant local hook/loop failure. The elasticity of the hook component 20 is attributable to elastic behavior of the hook backing 26. Unlike the hook backing 26, the individual hooks 24 have enough strength to remain engaged under tension.

Suitably, the hook backing 26 and loop backing 30 can each extend between about 50% and 200%, more suitably between about 70% and 170%, most suitably between about 100% and 150%. Desirably, the hook component 20 and the loop component 22 have equal strength and equal extensibility.

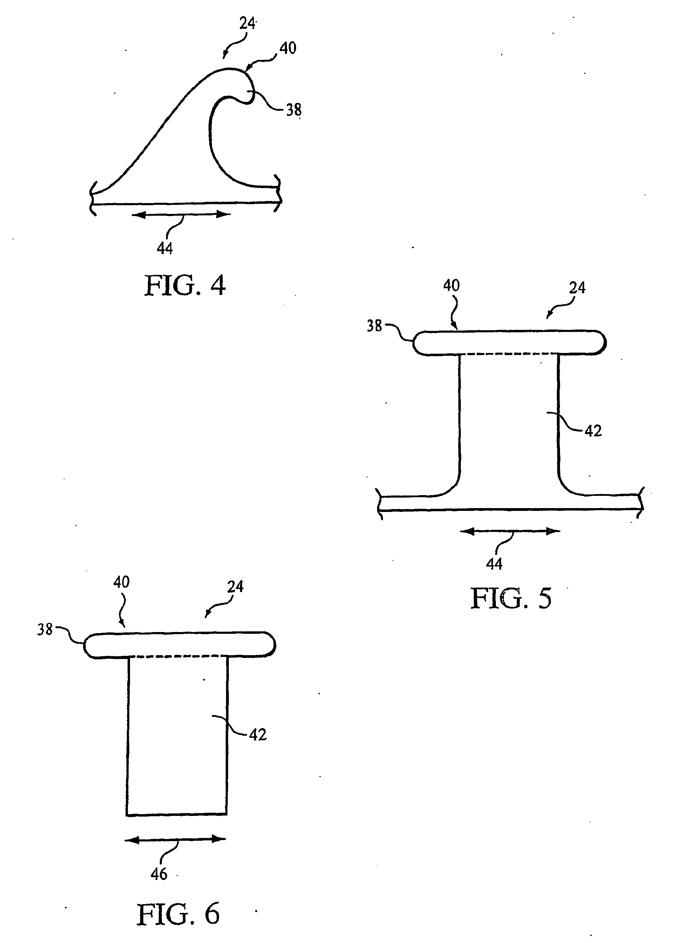

Virtually any hook shape can be used with the hook component 20. Suitably, the individual hooks 24 have an engagement portion 38 at a free end 40 of each hook 24. The engagement portion 38 can be J-shaped (Fig. 4), or a flat top (Figs. 5 and 6), or any other suitable shape. A hook 24 having a J-shaped engagement portion 38 is shown in Fig. 4 in a longitudinal direction. The flat-top hook 24 shown in Figs. 5 and 6 has a rounded engagement portion 38 with a flat top and can look the same in the longitudinal direction (Fig. 5) as in the transverse direction (Fig. 6), in which case a base portion 42 of the hook 24 is suitably round or square as viewed from above. Alternatively, the base portion 42 of the hook 24 can be oblong, rectangular, triangular, or any other suitable shape.

The longitudinal direction is indicated by an arrow 44 in Figs. 2, 4 and 5. The term "transverse direction" refers to a direction perpendicular to the longitudinal direction. The transverse direction is indicated by an arrow 46 in Figs. 1 and 6. In terms of a pant-like absorbent garment, the transverse direction of each fastening component located along a side of the garment is in a direction parallel to the wearer's waistline. Thus, the longitudinal direction of a fastening component on a pant-like absorbent garment is in a direction parallel to a wearer's backbone. For example, a diaper 60 including the fastening system 48 is shown in a stretched flat state, showing the surface of the diaper 60 that faces away from the wearer when the diaper 60 is worn, in Fig. 7, with arrow 44 indicating the longitudinal direction and arrow 46 indicating the transverse direction.

With reference to the diaper 60 in Fig. 7, the hook backing 26 attached to the diaper 60 is suitably stretchable in the transverse direction to adjust for expansion and contraction of the wearer's abdomen and hip circumference as the wearer's body flexes. If the hook backing 60 would not stretch, at least in the transverse direction, then the hook component 20 and the loop component 22 would be likely to become unfastened under shear forces exerted by the wearer's body as the body deforms the product. Therefore, both the hook backing 26 and the loop backing 30 of the fastening system 48 are suitably stretchable in the transverse direction or the longitudinal direction. Furthermore, the hook backing 26 and the loop backing 30 can be biaxially stretchable, thereby accommodating the wearer's body's movements in the transverse direction as well as the longitudinal direction. Areas 62 representing transverse stretchability in the hook backing 26 and the loop backing 30 are shown in Fig. 1. Similarly, areas 64 representing longitudinal stretchability in the hook backing 26 and the loop backing 30 are shown in Fig. 2.

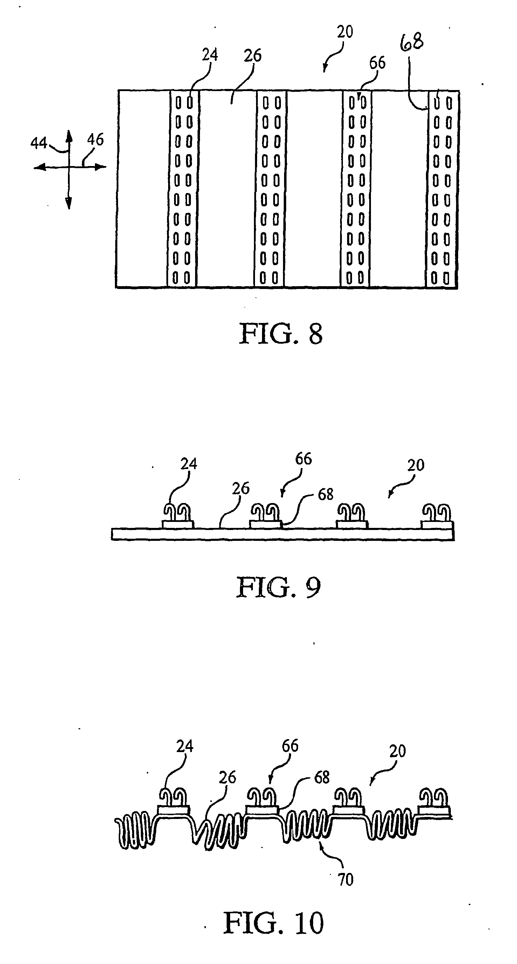

To further enhance the transverse stretch of the hook component 20, the individual hooks 24 may be embedded discontinuously in the hook backing 26. For example, the hooks 24 can be arranged in rows 66 on the hook backing 26, as shown in Fig. 8. The rows 66 are suitably parallel to the longitudinal direction, thereby not obstructing transverse stretch. Furthermore, the rows 66 of individual hooks can be embedded in strips of material 68, and the strips of material 68 can be bonded to the hook backing 26. Preferably, the strips of material 68 are bonded to the hook backing 26 using an adhesive, for example an elastomeric adhesive such as Findley H2525A, H2525 or H2096 of Findley Adhesives, Inc., of Wauwatosa, Wisconsin. Other bonding means well known to those having ordinary skill in the art may also be used to bond the strips of material 68 to the hook backing 26, including thermal bonding, ultrasonic bonding, and the like. Fig. 9 is a side view of Fig. 8 showing, more clearly, the strips of material 68 bonded to the hook backing 26. The strips of material 68 need not be stretchable in order to achieve the shear fastening strength resistance of the fastening system 68, but can be stretchable. More particularly, if the hook backing 26 is biaxially stretchable, then the strips of material 68 are suitably stretchable in at least the longitudinal direction.

In one embodiment, the hook component 20 includes at least two rows 66 of hooks 24 embedded in the hook backing 26, the rows 66 being parallel to the longitudinal direction. Between the rows 66, the hook backing 26 contains pleats 70, as shown in Fig. 10. These pleats 70 provide room for additional transverse direction expansion, in addition to the transverse stretch of the elastic hook backing 26. Methods of pleating materials are well-known in the art. The rows 66 of hooks 24 in this embodiment can either be attached to strips of material 68, which are then attached to the hook backing 26, or can be directly embedded in the hook backing material 26 to effectively create islands of hooks.

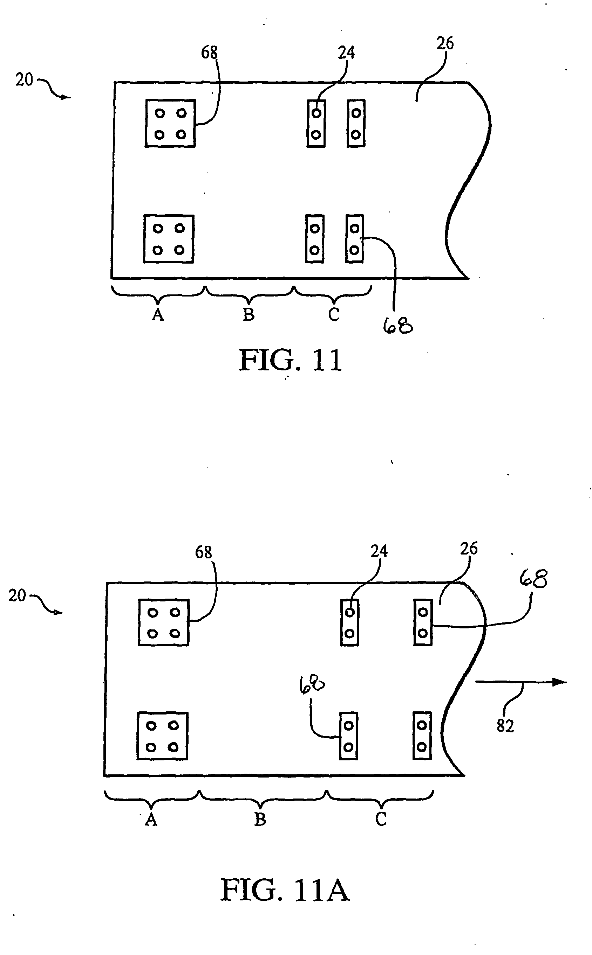

As noted above, rows of hooks 24 can be provided to facilitate stretching in the transverse direction. Also, groups of hooks 24' extending from strips of material 26 can be provided to facilitate a variety of stretching modes. Referring to Fig. 11, a hook component 20 is provided having material 68 that is attached to a biaxially extensible hook backing 26. The material 68 includes hooks 24 that extend from a surface of the material 68. The material 68 is preferably less extensible than the hook backing 26, as an example, the material is relatively non-extensible. As illustrated in Fig. 11A, when a force is applied in the direction indicated by arrow 82, the hook backing 26 extends. However, the amount of extension of the hook backing is affected by the less-extensible strips of material.

In region A, the hook component 20 is comparatively less extensible because the hook backing 26 is more dominated by the less-extensible material 68. In region B, the hook component is most extensible because the region includes no strips of material. In region C, the hook backing has an intermediate amount of extensibility because is it less dominated by the relatively non-extensible hook backing 26 compared to region A.

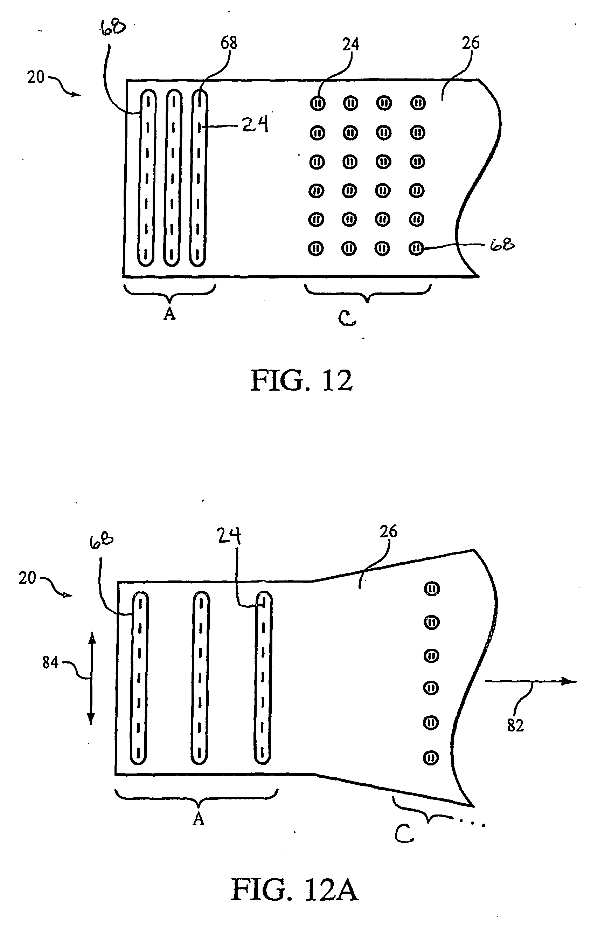

Referring now to Fig. 12, strips of material 68 are arranged to facilitate stretching in longitudinal and transverse directions. Region A contains strips of material 68 that extend in substantially parallel rows across the extensible hook backing 26. Where the strips 68 are substantially non-extensible, the rows act like beams that prevent extension of the substrate in the transverse direction forming a monoaxially extensible region. By contrast, region C contains strips of material that arranged in discrete, spaced-apart patches that allow extension of the hook backing in both the longitudinal and transverse directions.

Referring now to Fig. 12A, when forces are applied in the directions indicated by arrows 82 and 84, the hook backing extends. In region A, because the hook material is arranged in parallel rows that extend across the hook backing, the backing extends in the longitudinal direction (indicated by arrow 82). However, the hook backing in region A extends less in the transverse direction (indicated by arrow 84) due to the less-extensible material 68 that is arranged in transverse, parallel rows. In region C, the hook backing extends in both the transverse and longitudinal directions. This is because the less-extensible patches of material interferes less with the extension of the hook backing in both the transverse and longitudinal directions.

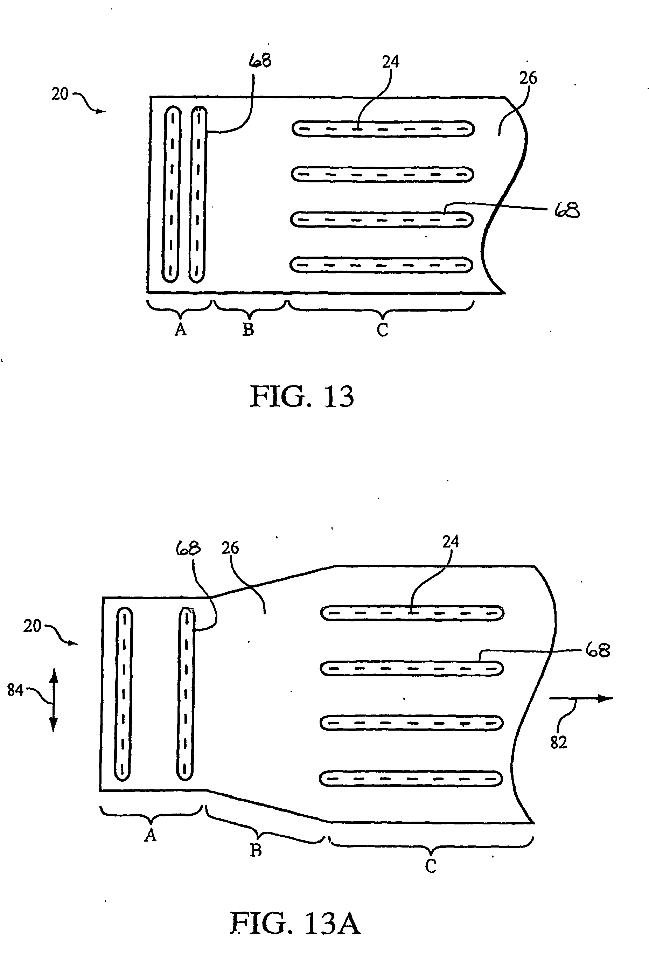

In a variation, illustrated by Fig. 13, a hook component 20 has a region A of longitudinal extensibility and a region C of transverse extensibility. Between regions A and C is a transition region B. In region A, material 68 that is less extensible than hook backing 26 is arranged in transverse rows allowing for longitudinal extensibility. In region C, the less extensible strips of material 68 are arranged in longitudinal rows allowing for transverse extensibility. As indicated by Fig. 13A, like the hook component of Fig. 12, the transverse rows of strips 68 interfere substantially with the extension of the hook backing in the transverse direction (indicated by arrow 84) within region A. Similarly, the longitudinal rows of strips 68 interfere substantially with the extension of the hook backing 26 in the longitudinal direction (indicated by arrow 82) within region C.

Although hook backing 26 is biaxially extensible, in regions A and C, the hook component is relatively monoaxially extensible due to the arrangement of the material 68. In region A, the hook component 20 extends in the longitudinal direction. In region C, the hook component extends transversely. Between region A and region C is transition region B that is biaxially extensible. Transition region B provides a transition from region A to the wider, extended region C.

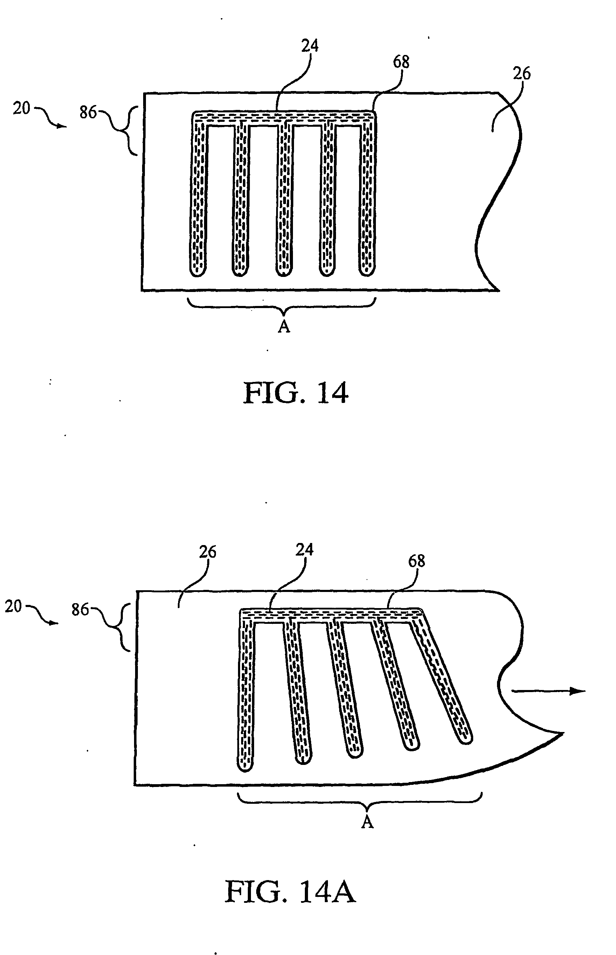

Referring now to Fig. 14, another hook component 20 is provided that includes less-extensible material 68. The material 68 is shaped to manipulate the extensibility of a biaxially extensible hook backing 26 in both the longitudinal and transverse directions within region A to create a region of asymmetric extensibility having an asymmetrical variance of longitudinal extensibility across the width of the hook component. Referring to Fig. 14A, the longitudinal extensibility of the hook component within region A changes across the width (i.e., in the transverse direction) of the hook component. Within section 86 of region A, the hook component 20 is relatively inextensible in the longitudinal direction due to the longitudinal beam 88 of material 68. Moving outwardly from the longitudinal beam in the transverse direction, the longitudinal extensibility of the hook backing increases. Also, within region A, the hook component of Figs. 13 and 13A is relatively inextensible in the transverse direction due to the transversely extending rows 90 of material 68 acting as beams. This is because the transverse rows 90 of material 68 interfere substantially with the extension of the hook backing in the transverse direction within the region.

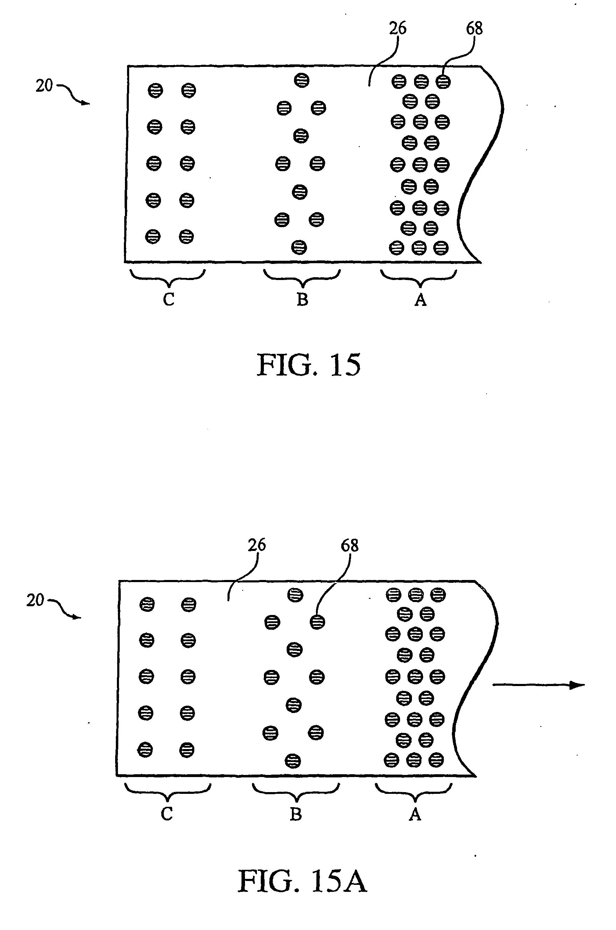

Referring now to Figs. 15 and 15A, less-extensible strips of material 68 are used to manipulate extensibility of a hook backing 26. Within region A, the strips of material are attached to the hook backing in a relatively dense arrangement, thus reducing the level of extensibility to near the extensibility level of the strips 68 (see Fig. 15A). Within region B, the arrangement of the strips 68 is less dense. As a result, region B of the hook component is more extensible than region A (see Fig. 15A). Similarly, region C has the lowest strip density and therefore has the highest level of longitudinal extensibility (see Fig. 15A).

Arrangement of the strips of material can be used to manipulate extensibility in a hook and loop fastening system. This can be accomplished by arranging less-extensible material on an extensible hook backing, as described above, or the material can be attached to extensible loop backing. Also, less-extensible material can be attached to both hook and loop extensible backings to form regions of the hook and loop system having particular stretching modes based on the arrangement of the less-extensible material. Regions of hook and loop backing having the less-extensible material can also cooperate to further manipulate the extensibility of the hook and loop system within a particular region. For example, a hook backing having a region including less-extensible material can overlap a loop backing region having less-extensible material. Although each of the hook and loop backings have a particular stretching mode due to a material arrangement, when hook components are engaged with loop components within the regions, the stretching modes cooperate to form a system stretching mode within the region.

A number of backing material can be employed. Instead of pleats 70, the hook backing material 26 can be creped, thereby providing room for additional transverse direction expansion. One technique of creping is taught, for example, in U.S. Patent 4,810,556, issued to Kobayashi et al. Essentially, the creping process involves coating a nonwoven fabric with a lubricant and then pressing the coated fabric between a drive roll and a plate having a rough sandpaper-like surface. The nonwoven fabric is crinkled in a wavelike fashion in the direction of movement of the fabric by the frictional force caused by the pressing.

The hook components 20 generally have between about 16 and about 620 hooks per square centimeter, suitably between about 124 and about 388 hooks per square centimeter, desirably between about 155 and about 310 hooks per square centimeter. The hooks 24 suitably have a height of from about 0.00254 centimeter (cm) to about 0.19 cm, suitably from about 0.0381 cm to about 0.0762 cm.

The hooks 24 are suitably molded or extruded from a thermoplastic polymer selected from polyamides, polyesters, polyolefins (e.g. polypropylene or polyethylene) or another suitable material. Likewise, the hook backing material 26, in addition to an elastic component described below, can be made of any of these or any other suitable materials since the hooks 24 and the hook backing 26 are generally produced from the same material in one process. Alternatively, the hooks 24 and the strips of material 68 can be co-formed from any of the listed materials, and the strips of material 68 can then be attached to an elastic hook backing material 26. The hook backing material 26 generally has a thickness in a range of between about 0.5 millimeter (mm) and about 5 mm, suitably in a range of between about 0.8 mm and 3 mm, with a basis weight in a range of from about 20 grams per square meter to about 70 grams per square meter. As mentioned, the hooks 24 can be spatially arranged in rows 66 or any other suitable discontinuous configuration on the hook backing 26.

Materials suitable for use in preparing the elastic hook backing 26 and the elastic loop backing 30 include diblock, triblock, tetrablock or other multi-block elastomeric copolymers such as olefinic copolymers, including styrene-isoprene-styrene, styrene-butadiene-styrene, styrene-ethylene/ butylene-styrene, or styrene-ethylene/propylene-styrene, which may be obtained from the Shell Chemical Company, under the trade designation KRATON^{®} elastomeric resin; polyurethanes, including those available from E. I. Du Pont de Nemours Co., under the trade name LYCRA^{®} polyurethane; polyamides, including polyether block amides available from Ato Chemical Company, under the trade name PEBAX^{®} polyether block amide; polyesters, such as those available from E. I. Du Pont de Nemours Co., under the trade name HYTREL^{®} polyester; and single-site or metallocene-catalyzed polyolefins having density less than about 0.89 grams/cc, available from Dow Chemical Co. under the trade name AFFINITY^{®}.

A number of block copolymers can be used to prepare the elastic hook backing 26 and the elastic loop backing 30. Such block copolymers generally comprise an elastomeric midblock portion B and a thermoplastic endblock portion A. The block copolymers may also be thermoplastic in the sense that they can be melted, formed, and resolidified several times with little or no change in physical properties (assuming a minimum of oxidative degradation).

Endblock portion A may comprise a poly(vinylarene), such as polystyrene. Midblock portion B may comprise a substantially amorphous polyolefin such as polyisoprene, ethylene/propylene polymers, ethylene/butylene polymers, polybutadiene, and the like, or mixtures thereof.

Suitable block copolymers include at least two substantially polystyrene endblock portions and at least one substantially ethylene/butylene mid-block portion. A commercially available example of such a linear block copolymer is a styrene-ethylene/butylene-styrene block copolymer, available from the Shell Chemical Company, under the trade designation KRATON^{®} G1657 elastomeric resin. Typical properties of KRATON^{®} G1657 elastomeric resin are reported to include a tensile strength of 3400 pounds per square inch (2x10⁶ kilograms per square meter), a 300 percent modulus of 350 pounds per square inch (1.4x10⁵ kilograms per square meter), an elongation of 750 percent at break, a Shore A hardness of 65, and a Brookfield viscosity, when at a concentration of 25 weight percent in a toluene solution, of about 4200 centipoise at room temperature. Another suitable elastomer, KRATON^{®} G2746, is a styrene butadiene block copolymer blended with tackifier and low density polyethylene.

Other suitable elastomeric polymers may also be used to make the elastic hook backing 26 and the elastic loop backing 30. These include, without limitation, elastomeric (single-site or metallocene catalyzed) polypropylene, polyethylene and other alpha-olefin homopolymers and copolymers, having density less than about 0.89 grams/cc; ethylene vinyl acetate copolymers; and substantially amorphous copolymers and terpolymers of ethylene-propylene, butene-propylene, and ethylene-propylene-butene.

Single-site catalyzed elastomeric polymers (for example, constrained geometry or metallocene-catalyzed elastomeric polymers) are relatively new, and are presently preferred. The single-site process for making polyolefins uses a single-site catalyst which is activated (i.e., ionized) by a co-catalyst.

Polymers produced using single-site catalysts have a narrow molecular weight distribution. "Narrow molecular weight distribution polymer" refers to a polymer that exhibits a molecular weight distribution of less than about 3.5. As is known in the art, the molecular weight distribution of a polymer is the ratio of the weight average molecular weight of the polymer to the number average molecular weight of the polymer. Methods of determining molecular weight distribution are described in the Encyclopedia of Polymer Science and Engineering, Volume 3, Pages 299-300 (1985). Polydispersities (M_{w}/Mₙ) of below 3.5 and even below 2 are possible for single-site produced polymers. These polymers also have a narrow short chain branching distribution when compared to otherwise similar Ziegler-Natta produced polymers.

It is also possible to use a single-site catalyst system to control the isotacticity of the polymer quite closely when stereo selective metallocene catalysts are employed. In fact, polymers have been produced having an isotacticity in excess of 99 percent. It is also possible to produce highly syndiotactic polypropylene using this system.

Commercial production of single-site catalyzed polymers is somewhat limited but growing. Such polymers are available from Exxon Chemical Company of Baytown, Texas under the trade name EXXPOL^{®} for polypropylene based polymers and EXACT^{®} for polyethylene based polymers. Dow Chemical Company of Midland, Michigan has polymers commercially available under the name ENGAGE^{®}. These materials are believed to be produced using non-stereo selective single-site catalysts. Exxon generally refers to their single-site catalyst technology as metallocene catalysts, while Dow refers to theirs as "constrained geometry" catalysts under the name INSITE^{®} to distinguish them from traditional Ziegler-Natta catalysts which have multiple reaction sites. Other manufacturers such as Fina Oil, BASF, Amoco, Hoechst and Mobil are active in this area and it is believed that the availability of polymers produced according to this technology will grow substantially in the next decade.

Regarding single-site catalyzed elastomeric polymers, U.S. Patent 5,204,429 to Kaminsky et al. describes a process which may produce elastic copolymers from cycloolefins and linear olefins using a catalyst which is a stereorigid chiral metallocene transition metal compound and an aluminoxane. The polymerization is carried out in an inert solvent such as an aliphatic or cycloaliphatic hydrocarbon such as toluene. The reaction may also occur in the gas phase using the monomers to be polymerized as the solvent. U.S. Patent 5,278,272 and 5,272,236, both to Lai et al., assigned to Dow Chemical and entitled "Elastic Substantially Linear Olefin Polymers" describe polymers having particular elastic properties. Dow also commercially produces a line of elastomeric polyolefins under the trade name ENGAGE^{®}.

The hook backing 26 and the loop backing 30 may also contain blends of elastic and inelastic polymers, or of two or more elastic polymers, provided that the blend exhibits elastic properties. Other suitable backing materials include films, stranded materials and "point unbonded" materials. Point unbonded materials are fabrics having continuous thermally bonded areas defining a plurality of discrete unbonded areas and are described in greater detail in U.S. Patent No. 5,858,515 issued January 12, 1999 to Stokes, et al. As used herein, the term "backing" can include hooks 24 or loops 28 as an integral part of the backing material.

The elastic hook backing 26 and loop backing 30 materials can be elastic nonwoven webs or laminates made of any of the listed polymers. The nonwoven webs can be stretch-thermal laminate (STL), neck-bonded laminate (NBL), reversibly necked laminate, or stretch-bonded laminate (SBL) material, using a stretchable polymer or a blend thereof. "Neck bonded laminate," or NBL, conventionally refers to a composite material having at least two layers in which one layer is a necked, non-elastic layer and the other layer is an elastic layer. The layers are joined together when the non-elastic layer is in an extended condition. "Reversibly necked laminate" conventionally refers to a composite material formed from at least one layer of material that has been treated while necked to impart memory to the material so that, when a force is applied to extend the material to its prenecked dimensions, the treated, necked portions will generally recover to their necked dimensions upon termination of the force. One form of treatment is the application of heat. "Stretch-bonded laminate," or SBL, conventionally refers to a composite material having at least two layers in which one layer is a gatherable layer and the other layer is an elastic layer. The layers are joined together when the elastic layer is in an extended condition so that upon relaxing the layers, the gatherable layer is gathered. Such a multilayer composite elastic material may be stretched to the extent that the nonelastic material gathered between the bond locations allows the elastic material to elongate. Methods of making such materials are well known to those skilled in the art and described in U.S. Patent 4,663,220 issued May 5, 1987 to Wisneski et al.; U.S. Patent 5,226,992 issued July 13, 1993 to Morman; and European Patent Application No. EP 0 217 032 published on April 8, 1987 in the names of Taylor et al.

Techniques for neck-bonding, i.e., laminating a neckable nonwoven web to an unstretched elastic film or other layer of material, are described in U.S. Patent 5,883,028, issued to Morman et al., which is incorporated by reference. The neckable nonwoven web may be a porous nonwoven material such as, for example, spunbonded web, meltblown web or bonded carded web. If the neckable material is a web of meltblown fibers, it may include meltblown microfibers. The neckable material may be made of fiber forming polymers such as, for example, polyolefins. Exemplary polyolefins include one or more of polypropylene, polyethylene, ethylene copolymers, propylene copolymers, and butene copolymers. Useful polypropylenes include, for example, polypropylene available from the Exxon Chemical Company under the trade designation Exxon 3445, and polypropylene available from Shell Chemical Company under the trade designation DX 5A09.

The neckable web may be a multilayer material having, for example, at least one layer of spunbonded web joined to at least one layer of meltblown web, bonded carded web or other suitable material. For example, neckable material may be a multilayer material having a first layer of spunbonded polypropylene having a basis weight from about 0.1 to about 8 ounces per square yard (osy) (about 3.4-270 grams/m², or gsm), a layer of meltblown polypropylene having a basis weight from about 0.1 to about 4 osy (3.4-135 gsm), and a second layer of spunbonded polypropylene having a basis weight of about 0.1 to about 8 osy (3.4-270 gsm). Alternatively, the neckable web may be a single layer of material such as, for example, a spunbonded web having a basis weight of from about 0.1 to about 10 osy (3.4-340 gsm) or a meltblown web having a basis weight of from about 0.1 to about 8 osy (3.4-270 gsm). The adjacent fibers of the web should be intermittently joined by interfiber bonding, using conventional techniques known in the art.

An elastic sheet may be joined to the neckable web when the latter is in the tensioned, necked state to form the neck-bonded laminate. Generally, the elastic film component of the neck-bonded laminate should be less than about 2 mils (50 microns) thick, preferably less than about 1 mil (25 microns) thick, more preferably less than about 0.5 mil (13 microns) thick, when the film and laminate are relaxed. In one embodiment, the strips of material 68 can be a neckable web and the hook backing 26 can be an elastic sheet joined to the neckable web to form a neckbonded laminate.

The individual loops 28 of the loop component 22 can be needled, stitched or otherwise connected to or projected through the loop backing material 30, which can suitably be made from a nonwoven material. The individual loops 28 thus connected can be made of yam or tow. Once the loops 28 have been formed, fibers forming the loops 28 can be anchored in place by bonding the fibers to the loop backing material 30 with heat and/or adhesives or any other suitable means. The individual loops 28 can alternatively be formed as an integral part of a fibrous nonwoven web such as a spunbond nonwoven web, or a staple fiber carded web. These nonwoven webs can be creped or crimped using processes known in the art, to form well-defined loop regions within their fiber structures. The polymers used to make the loop backing material 30 can be any of the elastic polymers listed above for the hook backing material 26. Suitably, the same elastic polymers can be used to form the loops 28.

The fastening system 48 is designed to elongate to a wearer's maximum product straining level to avoid significant hook/loop failure. The result is an elastic fastening system 48 including a hook component 20 and a loop component 22, each having elastic properties.

## Claims

1. A hook and loop fastener (48) comprising separate hook (20) and loop (22) components;
the hook component (20) including a hook backing (26) and a plurality of hooks (24) protruding from the hook backing, wherein the hook backing includes an extensible material; and
the loop component (22) including a loop backing (30) and a plurality of loops (28) protruding from it, wherein the loop backing includes an extensible material having relatively non-extensible resin disposed in spaced-apart regions of a broad surface of the loop backing (30).

2. The hook and loop fastener (48) of claim 1, wherein the hook component (20) further comprises a strip of material (68) between at least some of the hooks and the hook backing.

3. The hook and loop fastener (48) of claim 2, wherein the strips of material (68) are co-formed with the hooks.

4. The hook and loop fastener (48) of either of claims 2 or 3, wherein the strips of material (68) are stretchable in a longitudinal (44) direction and the hook backing (26) is biaxially stretchable.

5. The hook and loop fastener (48) of any of the above claims, wherein the hook backing (26) comprises an elastic nonwoven web, or a neck-bonded laminate.

6. The hook and loop fastener (48) of either of claims I or 5, wherein the extensible hook backing comprises:
an extensible sheet-form substrate (26) having first (A) and second (C) regions; and
a multiplicity of spaced-apart resin bases (68) disposed on a front side of the substrate (26), wherein the hooks (24) extend outwardly from and are integrally molded with the resin bases;
wherein bases in the first region (A) are arranged to provide different overall extensibility than in the second region (C).

7. The hook and loop fastener (48) of claim 6, wherein at least one of the extensible sheet-form substrate (26) and the extensible loop backing (30) are biaxially extensible, having an extensibility both in a first direction (82) and a second direction (84) perpendicular to the first direction (82).

8. The hook and loop fastener (48) of claim 1, wherein the relatively non-extensible resin is arranged to provide different overall extensibility in a first region than in a second region.

9. The hook and loop fastener (48) of claim 8, wherein the fastener elements (24) disposed in the first region (A) of the hook component (20) are engaged with loops (28) in the first region of the loop component (30).

10. The hook and loop fastener (48) of any of the above claims, wherein the loop backing (30) comprises a neck-bonded laminate, or an elastic nonwoven web, or an elastic woven web.

11. The hook and loop fastener (48) of any of the above claims, wherein the hook backing (26) can be stretched between 50% and 200%, particularly between 70% and 170%, more particularly between 100% and 150%.

12. The hook and loop fastener (48) of any of the above claims, wherein at least one of the extensible materials is creped.

13. The book and loop fastener (48) of any of the above claims, wherein the plurality of books (24) are aligned in at least two rows (66), the extensible material of the hook backing (26) including pleats (70) parallel to the rows (66) of books (24), the pleats (70) located between the rows of hooks.

14. The hook and loop fastener (48) of any of the above claims, wherein the hooks (24) include heads (38) that overhang the hook backing (26) in one or more discrete directions, or wherein the hooks (24) include heads (38) that overhang the hook backing (26) in all directions.

15. An absorbent article comprising:
an absorbent body (61);
and the hook and loop fastener (48) of any of the above claims, arranged on-the absorbent body to provide releasable fastening.

16. The absorbent article of claim 15, wherein the absorbent body is selected from the group consisting of a diaper, a feminine hygiene product, and an incontinence product.

17. A medical garment comprising:
a garment body; and
the hook and loop fastener (48) of any of claims 1-14, arranged on the garment body to provide releasable fastening.

18. A hook component (20) comprising:
an extensible, sheet-form substrate (26) having first (A) and second (C) regions; and
a multiplicity of spaced-apart resin bases disposed on a front side of the substrate, the spaced-apart resin bases (68) having integrally molded stems (42) extending therefrom;
wherein the bases in the first region (A) are arranged to provide different overall extensibility than in the second region (C).

19. The hook component (20) of claim 18, wherein the resin bases (68) are non-contiguous with each other.

20. The hook component (20) of either of claims 18 or 19, wherein the resin bases (68) are arranged to form areas of the substrate (26) having asymmetric extensibility.

21. The hook component (20) of any of claims 18-20, wherein the bases (68) extend in discrete, longitudinal or transverse rows.

22. The hook component (20) of any of claims 18-20, wherein the bases (68) form spaced-apart patches of hooks (24).

23. The hook component (20) of any of claims 18-22, wherein the substrate material is biaxially extensible having an extensibility both in a first direction (82) and a second direction (84) perpendicular to the first direction.

24. The hook component (20) of claim 23, wherein the bases (68) are arranged to limit the level of extensibility of the extensible substrate (26) more in one of the first (82) and second (84) directions than in the other direction in the first region (A).

25. The hook component (20) of either of claims 23 or 24, wherein the bases (68) are arranged to limit the level of extensibility of the extensible substrate in both the first (82) and second (84) directions in the first region (A).

26. The hook component (20) of any of claims 18-25, wherein the bases (68) are substantially non-extensible.

27. The hook component (20) of any of claims 18-26, wherein the extensible substrate (26) comprises an elastic nonwoven web, or an elastic woven web, or a neck-bonded laminate.

28. The hook component (20) of any of claims 18-27, wherein the extensible substrate can be stretched between about 50% and about 200%, preferably between about 70% and about 170%.

29. The hook component (20) of any of claims 18-28 further including head portions (38) extending radially from a distal end (40) of the stems (42).

30. The hook component (20) of any of claims 18-29, wherein the head portions (38) overhang the base (68) in one or more discrete directions, or wherein the head portions overhang the base in all directions.

31. A method of forming a hook and loop fastener product, the method comprising:
providing a sheet-form extensible first substrate (26) having first (A) and second (C) regions;
attaching a multiplicity of spaced-apart resin bases (68) on a front side of the substrate in the first region, the spaced-apart resin bases having fastener elements (24) including stems (42) extending outwardly from and integrally molded with the resin bases, to provide different overall extensibility in the first region than in the second region; and
engaging the fastener elements with matable elements (28) extending from a discrete second substrate.

32. The method of claim 31, wherein the matable elements are loops (28).

33. The method of either of claims 31 or 32, wherein the second substrate is extensible.

34. The method of any of claims 31-33, wherein the second substrate is biaxially extensible having an extensibility on a first direction (82) and a second direction (84), perpendicular to the first direction.

35. The method of claim 34 further including attaching a multiplicity of discrete, spaced-apart material on a broad surface of the second substrate in a first region to provide a different overall extensibility in the first region relative to a second region of the second substrate.

36. The method of any of claims 31-35, wherein the fastener elements (24) include head portions (38) that overhang the base (68) in one or more discrete directions, or wherein the fastener elements include head portions that overhang the base in all directions.

37. The method of any of claims 31-36, wherein the resin bases (68) are non-contiguous with each other.

38. The method of any of claims 31-37, wherein the resin bases (68) are arranged to form areas of the substrate (26) having asymmetric extensibility.

39. The method of any of claims 31-38, wherein the matable elements (28) are nonwoven fibers.

## Patentansprüche

1. Klettverschluss (48), aufweisend getrennte Bestandteile aus Haken (20) und Schlingen (22);
wobei der Hakenbestandteil (20) einen Hakenträger (26) und eine Mehr- bzw. Vielzahl vom Hakenträger vorstehende Haken (24) einschließt, wobei der Hakenträger ein dehnbares Material einschließt; und
wobei der Schlingenbestandteil (22) einen Schlingenträger (30) und eine Mehr- bzw. Vielzahl von davon vorstehender Schlingen (28) einschließt, wobei der Schlingenträger ein dehnbares Material mit relativ undehnbarem Harz, das in beabstandeten Bereichen einer breiten Oberfläche der Schlingenträger (30) angeordnet ist, einschließt.

2. Klettverschluss (48) nach Anspruch 1, wobei der Hakenbestandteil (20) ferner einen Materialstreifen (68) zwischen mindestens einigen der Haken und dem Hakenträger aufweist.

3. Klettverschluss (48) nach Anspruch 2, wobei die Materialstreifen (68) gemeinsam mit den Haken gebildet sind.

4. Klettverschluss (48) nach einem der Ansprüche 2 oder 3, wobei die Materialstreifen (68) in eine Längsrichtung (44) streckbar sind und der Hakenträger (26) biaxial streckbar ist.

5. Klettverschluss (48) nach einem der vorstehenden Ansprüche, wobei der Hakenträger (26) eine elastische, nicht gewebte Bahn bzw. einen Vliesstoff oder ein halsgebundenes Laminat aufweist.

6. Klettverschluss (48) nach einem der Ansprüche 1 oder 5, wobei der dehnbare Hakenträger Folgendes aufweist:
ein dehnbares lagenförmiges Substrat (26) mit ersten (A) und zweiten (C) Bereichen; und
eine Vielzahl von an einer Vorderseite des Substrats (26) angeordneten beabstandeten Harzsockeln (68), wobei sich die Haken (24) von den Harzsockeln nach außen erstrecken und damit integral gebildet sind;
wobei Sockel im ersten Bereich (A) zum Bereitstellen einer anderen Gesamtdehnbarkeit als im zweiten Bereich (C) angeordnet sind.

7. Klettverschluss (48) nach Anspruch 6, wobei mindestens eines des dehnbaren lagenförmigen Substrats (26) und des dehnbaren Schleifenträgers (30) mit einer Dehnbarkeit, sowohl in eine erste Richtung (82) als auch in eine zweite Richtung (84), die rechtwinklig zur ersten Richtung (82) liegt, biaxial dehnbar ist.

8. Klettverschluss (48) nach Anspruch 1, wobei das relativ undehnbare Harz zum Bereitstellen einer anderen Gesamtdehnbarkeit in einem ersten Bereich als in einem zweiten Bereich angeordnet ist.

9. Klettverschluss (48) nach Anspruch 8, wobei die im ersten Bereich (A) des Hakenbestandteils (20) angeordneten Befestigungselemente (24) mit den Schlingen (28) im ersten Bereich des Schlingenbestandteils (30) in Eingriff stehen.

10. Klettverschluss (48) nach einem der vorstehenden Ansprüche, wobei der Schlingenträger (30) ein halsgebundenes Laminat oder eine elastische, nicht gewebte Bahn bzw. einen Vliesstoff oder ein elastisches Gewebe aufweist.

11. Klettverschluss (48) nach einem der vorstehenden Ansprüche, wobei der Hakenträger (26) zwischen 50 % und 200 %, insbesondere zwischen 70 % und 170 %, insbesondere zwischen 100 % und 150 % gestreckt werden kann.

12. Klettverschluss (48) nach einem der vorstehenden Ansprüche, wobei mindestens eines der dehnbaren Materialien gekreppt ist.

13. Klettverschluss (48) nach einem der vorstehenden Ansprüche, wobei die Mehr- bzw. Vielzahl Haken (24) in mindestens zwei Reihen (66) ausgerichtet sind, das dehnbare Material des Hakenträgers (26) Falten (70) einschließt, die parallel zu den Reihen (66) der Haken (24) liegen, die Falten (70) zwischen den Hakenreihen lokalisiert sind.

14. Klettverschluss (48) nach einem der vorstehenden Ansprüche, wobei die Haken (24) Köpfe (38) einschließen, die in eine oder mehrere getrennte Richtungen über den Hakenträger (26) ragen oder wobei die Haken (24) Köpfe (38) einschließen, die in alle Richtungen über den Hakenträger (26) ragen.

15. Saugfähiger Gegenstand aufweisend:
einen saugfähigen Körper (61);
und den Klettverschluss (48) nach einem der vorstehenden Ansprüche, der zum Bereitstellen einer lösbaren Befestigung auf dem saugfähigen Körper angeordnet ist.

16. Saugfähiger Gegenstand nach Anspruch 15, wobei der saugfähige Körper ausgewählt ist aus der Gruppe bestehend aus einer Windel, einem Hygieneprodukt für Frauen und einem Inkontinenzprodukt.

17. Medizinisches Kleidungsstück, aufweisend:
einen Kleidungsstückkörper; und
den Klettverschluss (48) nach einem der Ansprüche 1-14, der zum Bereitstellen einer lösbaren Befestigung auf dem Kleidungsstückkörper angeordnet ist.

18. Hakenbestandteil (20), aufweisend:
ein dehnbares lagenförmiges Substrat (26) mit ersten (A) und zweiten (C) Bereichen; und
einer Vielzahl an auf einer Vorderseite des Substrats angeordneten beabstandeten Harzsockeln, wobei die beabstandeten Harzsockel (68) sich davon erstreckende integral gebildete Stiele (42) aufweisen;
wobei die Sockel im ersten Bereich (A) zum Bereitstellen einer anderen Gesamtdehnbarkeit als im zweiten Bereich (C) angeordnet sind.

19. Hakenbestandteil (20) nach Anspruch 18, wobei die Harzsockel (68) nicht aneinander angrenzen.

20. Hakenbestandteil (20) entweder nach Anspruch 18 oder 19, wobei die Harzsockel (68) zum Bilden von Bereichen des Substrats (26) mit unsymmetrischer Dehnbarkeit angeordnet sind.

21. Hakenbestandteil (20) nach einem der Ansprüche 18-20, wobei sich die Sokkel (68) in getrennte, Längs- oder Querreihen erstrecken.

22. Hakenbestandteil (20) nach einem der Ansprüche 18-20, wobei die Sockel (68) beabstandete Hakenfelder (24) bilden.

23. Hakenbestandteil (20) nach einem der Ansprüche 18-22, wobei das Substratmaterial mit einer Dehnbarkeit, sowohl in eine erste Richtung (82) als auch in eine zweite Richtung (84), die rechtwinklig zur ersten Richtung liegt, biaxial dehnbar ist.

24. Hakenbestandteil (20) nach Anspruch 23, wobei die Sockel (68) zum Beschränken des Dehnbarkeitsgrads des dehnbaren Substrats (26) mehr in eine der ersten (82) und zweiten (84) Richtung, als in die andere Richtung im ersten Bereich (A) angeordnet sind.

25. Hakenbestandteil (20), nach einem der Ansprüche 23 oder 24, wobei die Sokkel (68) zum Beschränken des Dehnbarkeitsgrads des dehnbaren Substrats sowohl in die erste (82) als auch in die zweite (84) Richtung im ersten Bereich (A) angeordnet sind.

26. Hakenbestandteil (20) nach einem der Ansprüche 18-25, wobei die Sockel (68) im Wesentlichen nicht dehnbar sind.

27. Hakenbestandteil (20) nach einem der Ansprüche 18-26, wobei das dehnbare Substrat (26) eine elastische, nicht gewebte Bahn bzw. einen Vliesstoff oder ein elastisches Gewebe oder ein halsgebundenes Laminat aufweist.

28. Hakenbestandteil (20) nach einem der Ansprüche 18-27, wobei das dehnbare Substrat (26) zwischen etwa 50 % und etwa 200 %, vorzugsweise zwischen etwa 70 % und etwa 170 % gestreckt werden kann.

29. Hakenbestandteil (20) nach einem der Ansprüche 18-28, ferner aufweisend Kopfteile (38), die sich von einem entfernten Ende (40) der Stiele (42) radial erstrecken.

30. Hakenbestandteil (20) nach einem der Ansprüche 18-29, wobei die Kopfteile (38) in eine oder mehrere getrennte Richtungen über den Sockel (68) ragen oder wobei die Kopfteile in alle Richtungen über den Sockel ragen.

31. Verfahren zum Bilden eines Klettverschlussprodukts, wobei das Verfahren Folgendes aufweist:
Bereitstellen eines lagenförmigen dehnbaren ersten Substrats (26) mit ersten (A) und zweiten (C) Bereichen;
Anbringen einer Vielzahl von beabstandeten Harzsockeln (68) an einer Vorderseite des Substrats im ersten Bereich, wobei die beabstandeten Harzsokkel Befestigungselemente (24) aufweisen, einschließend Stiele (42), die sich von den Harzsockeln nach außen erstrecken und damit integral sind, um im ersten Bereich eine andere Gesamtdehnbarkeit als im zweiten Bereich bereitzustellen; und
Eingreifen der Befestigungselemente in zusammenfügbare Elemente (28), die sich von einem getrennten zweiten Substrat erstrecken.

32. Verfahren nach Anspruch 31, wobei die zusammenfügbaren Elemente Schlingen (28) sind.

33. Verfahren entweder nach Anspruch 31 oder 32, wobei das zweite Substrat dehnbar ist.

34. Verfahren nach einem der Ansprüche 31-33, wobei das zweite Substrat mit einer Dehnbarkeit in eine erste Richtung (82) und eine zweite Richtung (84), die rechtwinklig zur ersten Richtung liegt, biaxial dehnbar ist.

35. Verfahren nach Anspruch 34, ferner einschließend das Anbringen einer Vielzahl von getrennten, beabstandeten Materialien auf einer breiten Oberfläche des zweiten Substrats in einem ersten Bereich, um im ersten Bereich in Bezug auf einen zweiten Bereich eine andere Gesamtdehnbarkeit des zweiten Substrats bereitzustellen.

36. Verfahren nach einem der Ansprüche 31-35, wobei die Befestigungselemente (24) Kopfteile (38) einschließen, die in eine oder mehrere getrennte Richtungen über den Sockel (68) ragen oder wobei die Befestigungselemente Kopfteile einschließen, die in alle Richtungen über den Sockel ragen.

37. Verfahren nach einem der Ansprüche 31-36, wobei die Harzsockel (68) nicht aneinander angrenzen.

38. Verfahren nach einem der Ansprüche 31-37, wobei die Harzsockel (68) zum Bilden von Bereichen des Substrats (26) mit unsymmetrischer Dehnbarkeit angeordnet sind.

39. Verfahren nach einem der Ansprüche 31-38, wobei die zusammenfügbaren Elemente (28) nicht gewebte Fasern bzw. Vliesstofffasern sind.

## Revendications

1. Fermeture auto-agrippante (48) comprenant des composants séparés de crochet (20) et de boucle (22) ;
le composant de crochet (20) comprenant un support de crochet (26) et une pluralité de crochets (24) faisant saillie à partir du support de crochet, dans lequel le support de crochet comprend un matériau extensible ;
le composant de boucle (22) comprenant un support de boucle (30) et une pluralité de boucles (28) faisant saillie à partir de celui-ci, dans lequel le support de boucle comprend un matériau extensible présentant une résine relativement non extensible disposée dans des régions espacées les unes des autres d'une large surface du support de boucle (30).

2. Fermeture auto-agrippante (48) selon la revendication 1, dans lequel le composant de crochet (20) comprend en outre une bande de matériau (68) entre au moins certains des crochets et le support de crochet.

3. Fermeture auto-agrippante (48) selon la revendication 2, dans lequel les bandes de matériau (68) sont formées conjointement aux crochets.

4. Fermeture auto-agrippante (48) selon l'une des revendications 2 ou 3, dans lequel les bandes de matériau (68) sont étirables dans une direction longitudinal (44) et le support de crochet (26) est étirable de façon biaxiale.

5. Fermeture auto-agrippante (48) selon l'une quelconque des revendications précédentes, dans lequel le support de crochet (26) comprend une bande non tissée élastique, ou un stratifié lié par striction.

6. Fermeture auto-agrippante (48) selon l'une des revendications 1 ou 5, dans lequel le support de crochet extensible comprend :
un substrat en feuille extensible (26) présentant des première (A) et seconde (C) régions ;
et une multiplicité de bases en résine éloignées les unes des autres (68) disposées sur un côté avant du substrat (26), dans lequel les crochets (24) s'étendent vers l'extérieur à partir des bases en résine et sont moulés d'un seul tenant avec celles-ci ;
dans lequel des bases dans la première région (A) sont agencées pour fournir une extensibilité générale différente de la seconde région (C).

7. Fermeture auto-agrippante (48) selon la revendication 6, dans lequel au moins un parmi le substrat en feuille extensible (26) et le support de boucle extensible (30) sont extensibles de façon biaxiale, présentant une extensibilité à la fois dans une première direction (82) et une seconde direction (84) perpendiculaire à la première direction (82).

8. Fermeture auto-agrippante (48) selon la revendication 1, dans lequel la résine relativement non extensible est agencée pour fournir une extensibilité générale dans une première région différente d'une seconde région.

9. Fermeture auto-agrippante (48) selon la revendication 8, dans lequel les éléments de fermeture (24) disposés dans la première région (A) du composant de crochet (20) sont en prise avec des boucles (28) dans la première région du composant de boucle (30).

10. Fermeture auto-agrippante (48) selon l'une quelconque des revendications précédentes, dans lequel le support de boucle (30) comprend un stratifié lié par striction, ou une bande non tissée élastique, ou une bande tissée élastique.

11. Fermeture auto-agrippante (48) selon l'une quelconque des revendications précédentes, dans lequel le support de crochet (26) peut être étiré entre 50 % et 200 %, particulièrement entre 70 % et 170 %, plus particulièrement entre 100 % et 150 %.

12. Fermeture auto-agrippante (48) selon l'une quelconque des revendications précédentes, dans lequel au moins un des matériaux extensibles est crêpé.

13. Fermeture auto-agrippante (48) selon l'une quelconque des revendications précédentes, dans lequel la pluralité de crochets (24) sont alignés dans au moins deux rangées (66), le matériau extensible du support de crochet (26) comprenant des plis (70) parallèles aux rangées (66) de crochets (24), les plis (70) positionnés entre les rangées de crochets.

14. Fermeture auto-agrippante (48) selon l'une quelconque des revendications précédentes, dans lequel les crochets (24) comprennent des têtes (38) qui surplombent le support de crochet (26) dans une ou plusieurs directions distinctes, ou dans lequel les crochets (24) comprennent des têtes (38) qui surplombent le support de crochet (26) dans toutes les directions.

15. Article absorbant comprenant:
un corps absorbant (61) ;
et la fermeture auto-agrippante (48) selon l'une quelconque des revendications précédentes, agencée sur le corps absorbant pour fournir une fermeture libérable.

16. Article absorbant selon la revendication 15, dans lequel le corps absorbant est sélectionné parmi le groupe constitué d'une couche, un produit d'hygiène féminine, et un produit d'incontinence.

17. Vêtement médical comprenant:
un corps de vêtement ;
et la fermeture auto-agrippante (48) selon l'une quelconque des revendications 1 à 14, agencée sur le corps de vêtement pour fournir une fermeture libérable.

18. Composant de crochet (20) comprenant:
un substrat en feuille extensible (26) présentant des première (A) et seconde (C) régions ; et
une multiplicité de bases en résine éloignées les unes des autres disposées sur un côté avant du substrat, les bases en résine éloignées les unes des autres (68) présentant des tiges moulées d'un seul tenant (42) s'étendant à partir de celles-ci ;
dans lequel les bases dans la première région (A) sont agencées pour fournir une extensibilité générale différente de la seconde région (C).

19. Composant de crochet (20) selon la revendication 18, dans lequel les bases en résine (68) sont non contiguës les unes aux autres.

20. Composant de crochet (20) selon l'une des revendications 18 ou 19, dans lequel les bases en résine (68) sont agencées pour former des zones du substrat (26) présentant une extensibilité asymétrique.

21. Composant de crochet (20) selon l'une quelconque des revendications 18 à 20, dans lequel les bases (68) s'étendent dans des rangées distinctes longitudinales ou transversales.

22. Composant de crochet (20) selon l'une quelconque des revendications 18 à 20, dans lequel les bases (68) forment des pièces de crochets éloignées les unes des autres (24).

23. Composant de crochet (20) selon l'une quelconque des revendications 18 à 22, dans lequel le substrat matériau est extensible de façon biaxiale présentant une extensibilité à la fois dans une première direction (82) et une seconde direction (84) perpendiculaire à la première direction.

24. Composant de crochet (20) selon la revendication 23, dans lequel les bases (68) sont agencées pour limiter le niveau d'extensibilité du substrat extensible (26) davantage dans une des première (82) et seconde (84) directions que dans l'autre direction dans la première région (A).

25. Composant de crochet (20) selon l'une des revendications 23 ou 24, dans lequel les bases (68) sont agencées pour limiter le niveau d'extensibilité du substrat extensible dans les première (82) et seconde (84) directions dans la première région (A).

26. Composant de crochet (20) selon l'une quelconque des revendications 18 à 25, dans lequel les bases (68) sont sensiblement non extensibles.

27. Composant de crochet (20) selon l'une quelconque des revendications 18 à 26, dans lequel le substrat extensible (26) comprend une bande non tissée élastique, ou une bande tissée élastique, ou un stratifié lié par striction.

28. Composant de crochet (20) selon l'une quelconque des revendications 18 à 24, dans lequel le substrat extensible peut être étiré entre environ 50 % et environ 200 %, de préférence entre environ 70 % et environ 170 %.

29. Composant de crochet (20) selon l'une quelconque des revendications 18 à 28, comprenant en outre des parties de tête (38) s'étendant de façon radiale à partir d'une extrémité distale (40) des tiges (42).

30. Composant de crochet (20) selon l'une quelconque des revendications 18 à 29, dans lequel les parties de tête (38) surplombent la base (68) dans une ou plusieurs directions distinctes, ou dans lequel les parties de tête surplombent la base dans toutes les directions.

31. Procédé de formage d'un produit de fermeture auto-agrippante, le procédé comprenant les étapes consistant à :
fournir un premier substrat extensible en feuille (26) présentant des première (A) et seconde (C) régions ;
fixer une multiplicité de bases en résine éloignées les unes des autres (68) sur un côté avant du substrat dans la première région, les bases en résine éloignées les unes des autres comportant des éléments de fermeture (24) comprenant des tiges (42) s'étendant vers l'extérieur à partir des bases en résine et moulées d'un seul tenant avec celles-ci, pour fournir une extensibilité générale dans la première région différente de la seconde région ; et
mettre en prise les éléments de fermeture avec des éléments pouvant être accouplés (28) s'étendant à partir d'un second substrat distinct.

32. Procédé selon la revendication 31, dans lequel les éléments pouvant être accouplés sont des boucles (28).

33. Procédé selon l'une des revendications 31 ou 32, dans lequel le second substrat est extensible.

34. Procédé selon l'une quelconque des revendications 31 à 33, dans lequel le second substrat est extensible de façon biaxiale présentant une extensibilité sur une première direction (82) et une seconde direction (84) perpendiculaire à la première direction.

35. Procédé selon la revendication 34, comprenant en outre l'étape consistant à fixer une multiplicité de matériaux distincts éloignés les uns des autres sur une large surface du second substrat dans une première région pour fournir une extensibilité générale différente dans la première région par rapport à une seconde région du second substrat.

36. Procédé selon l'une quelconque des revendications 31 à 35, dans lequel les éléments de fermeture (24) comprennent des parties de tête (38) qui surplombent la base (68) dans une ou plusieurs directions distinctes, ou dans lequel les éléments de fermeture comprennent des parties de tête qui surplombent la base dans toutes les directions.

37. Procédé selon l'une quelconque des revendications 31 à 36, dans lequel les bases en résine (68) sont non contiguës les unes aux autres.

38. Procédé selon l'une quelconque des revendications 31 à 37, dans lequel les bases en résine (68) sont agencées pour former des zones du substrat (26) présentant une extensibilité asymétrique.

39. Procédé selon l'une quelconque des revendications 31 à 38, dans lequel les éléments pouvant être accouplés (28) sont des fibres non tissées.
